# FASCICULE DE BREVET EUROPEEN

(11) **EP 0 966 951 B1**
(45) Date de publication et mention de la délivrance du brevet: **24.10.2001**
(21) Numéro de dépôt: 99401356.3
(22) Date de dépôt: 04.06.1999
(51) Int. Cl.: A61K 7/13

(54) **Composition tinctoriale contenant du 1,8-bis-(2,5-diaminophénoxy)-3,6-dioxaoctane, une base d'oxydation additionnelle et un coupleur, et procédé de teinture**
Färbemittel das 1,8-Bis-(2,5-diaminophenoxy)-3,6-dioxaoctan, ein Oxidierungsmittel und einen Kuppler enthält, und Verfahren zur Färbung
Dye composition containing 1,8-bis-(2,5-diaminophenoxy)-3,6-dioxaoctane, an additional oxdation base and a coupler, and dyeing process

(30) Priorité: 19.06.1998 FR 9807793
(43) Date de publication de la demande: 29.12.1999
(73) Titulaire: L'OREAL, 75008 Paris (FR)
(72) Inventeur: Audousset, Marie-Pascale, 92600 Asnieres (FR)
(74) Mandataire: Goulard, Sophie

(56) Documents cités:
- WO-A-92/13824
- WO-A-97/49378
- DE-A- 4 314 317
- DE-A- 4 335 623
- DE-A- 4 335 628
- DE-A- 19 630 275

## Description

L'invention a pour objet une composition pour la teinture d'oxydation des fibres kératiniques contenant une première base d'oxydation choisie parmi le 1,8-bis-(2,5-diaminophénoxy)-3,6-dioxaoctane, et ses sels d'addition avec un acide, au moins une deuxième base d'oxydation sélectionnée et au moins un coupleur ; ainsi que le procédé de teinture d'oxydation mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bis-phénylalkylènediamines ou bien encore des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, en particulier dans la demande de brevet WO 92/13824 d'utiliser à titre de base d'oxydation et éventuellement en présence d'un coupleur, des 2,5-diaminophénoxy-oxaalcanes. Cependant les colorations obtenues en mettant en oeuvre ces bases d'oxydation ne sont pas entièrement satisfaisantes, notamment en ce qui concerne la puissance des colorations obtenues et leur résistance aux différents traitements que peuvent subir les cheveux. Les colorations obtenues présentent en particulier une résistance insuffisante à l'action de la lumière.

Or, la Demanderesse vient maintenant de découvrir, de façon totalement inattendue et surprenante, que l'association du 1,8-bis-(2,5-diaminophénoxy)-3,6-dioxaoctane, et/ou d'au moins un de ses sels d'addition avec un acide, avec au moins une deuxième base d'oxydation convenablement sélectionnée et au moins un coupleur permettait d'obtenir des colorations puissantes présentant de plus des propriétés de résistance améliorées vis à vis des diverses agressions que peuvent subir les cheveux (shampooings, lumière, intempéries, ondulations permanentes, transpiration, frottements).

Ces découvertes sont à la base de la présente invention.

L'invention a donc pour premier objet une composition pour la teinture d'oxydation des fibres kératiniques, et en particulier des fibres kératiniques humaines telles que les cheveux, caractérisée par le fait qu'elle contient, dans un milieu approprié pour la teinture :
- du 1,8-bis-(2,5-diaminophénoxy)-3,6-dioxaoctane et/ou au moins un de ses sels d'addition avec un acide, à titre de première base d'oxydation :
- au moins une base d'oxydation additionnelle choisie parmi la paraphénylènediamine, la paratoluylènediamine, la N,N-bis-(β-hydroxyéthyl)-paraphénylènediamine, la 2-(β-hydroxyéthyl) - paraphénylènediamine, la 2,6-diméthyl-paraphènylènediamine, la 2-isopropyl-paraphénylènediamine, la 2-chloro - paraphénylènediamine, la N-phényl - paraphénylènediamine, la 4,4'-diamino- diphénylamine, la N-méthoxyéthyl- paraphénylènediamine, la 2-n-propyl-paraphénylènediamine, le 4-aminophénol, le N-méthyl 4-aminophénol, le 2-hydroxyméthyl-4-aminophénol, le 3-méthyl-4-aminophénol, le 2-aminométhyl-4-aminophénol, le 2-(β-hydroxyéthylaminométhyl)-4-aminophénol, le 2-méthoxy-4-aminophénol, le 2-méthoxyméthyl-4-aminophénol
- et au moins un coupleur.

Comme indiqué précédemment, la composition tinctoriale conforme à l'invention conduit à des colorations puissantes qui présentent de plus d'excellentes propriétés de résistance vis à vis de l'action des différents agents extérieurs (lumière, intempéries, lavage, ondulation permanente, transpiration, frottements).

Ces propriétés sont particulièrement remarquables en ce qui concerne la résistance des colorations obtenues vis à vis de la lumière,

La nature du ou des coupleurs pouvant être utilisés dans la composition tinctoriale conforme à l'invention n'est pas critique. Ils peuvent être choisis parmi les coupleurs classiquement utilisés pour ia teinture des fibres kératiniques et parmi lesquels on peut notamment citer les métaphénylènediamines, les métaaminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 5-amino-2-méthyl phénol, le 5-N-(β-hydroxyéthyl)amino-2-méthyl-phénol, le 3-amino-phénol, le 1,3-dihydroxy- benzène, le 1,3-dihydroxy-2-méthyl-benzène, le 4-chloro-1,3-dihydroxy-benzène, le 2,4-diamino-1-(β-hydroxyéthyloxy)-benzène, le 2-amino 4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxyindole, le 4-hydroxy-indole, le 4-hydroxy-N-méthyl-indole, la 6-hydroxy-indoline, la 6-hydroxybenzomorpholine, la 2,6-dihydroxy-4-méthyl-pyridine, le 1-H-3-méthylpyrazole-5-one, le 1-phényl-3-méthyl-pyrazole-5-one, et leurs sels d'addition avec un acide.

D'une manière générale, les sels d'addition avec un acide utilisables dans le cadre de l'invention (bases d'oxydation et coupleurs) sont notamment choisis parmi les chlorhydrates, les bromhydrates, les sulfates, les citrates, les succinates, les tartrates, les lactates et les acétates.

Le 1,8-bis-(2,5-diaminophénoxy)-3,6-dioxaoctane et/ou le ou ses sels d'addition avec un acide représentent de préférence de 0,0005 à 12% en poids environ du poids total de la composition tinctoriale, et encore plus préférentiellement de 0,005 à 6 % en poids environ de ce poids.

La ou les bases d'oxydation additionnelles conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids

Le ou les coupleurs représentent de préférence de 0,0001 à 10 % en poids environ du poids total de la composition tinctoriale et encore plus préférentiellement de 0,005 à 5 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylénegiycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 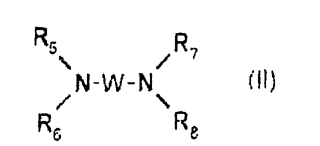 dans laquelle W est un reste propylène éventuellement substitué par un groupement hydroxyle ou un radical alkyle en C₁-C₆; R₅, R₆, R₇ et R₈, identiques ou différents, représentent un atome d'hydrogène; un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, **par exemple** sous forme de liquides, de crèmes, de gels, ou sous toute autre forme appropriée pour réaliser une teinture des fibres kératiniques, et notamment des cheveux humains

L'invention a également pour objet un procédé de teinture d'oxydation des fibres kératiniques et en particulier des fibres kératiniques humaines telles que les cheveux mettant en oeuvre la composition tinctoriale définie précédemment.

Ce procédé comprend l'application sur les fibres d'une composition tinctoriale définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange, de préférence au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques et parmi lesquels on peut citer le peroxyde d'hydrogène, le peroxyde d'urée, les bromates de métaux alcalins, les persels tels que les perborates et persulfates, et les enzymes parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le pH de la composition oxydante renfermant l'agent oxydant défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.
La composition oxydante définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, **par exemple** sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture à plusieurs compartiments dont un premier compartiment renferme la composition tinctoriale définie ci-dessus et un second compartiment renferme la composition oxydante définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples suivants sont destinés à illustrer l'invention sans pour autant présenter un caractère limitatif.

### EXEMPLES

### EXEMPLES 1 à 6 DE TEINTURE EN MILIEU ALCALIN

On a préparé les compositions tinctoriales conformes à l'invention suivantes (teneurs en grammes) :

| **EXEMPLE** | **1** | **2** | **3** | **4** | **5** | **6** |
|---|---|---|---|---|---|---|
| 1,8-bis-(2,5-diaminophénoxy)-3,6-dioxaoctane, tétrachlorhydrate, monohydrate | 0,39 | 0,39 | 0,39 | 0,39 | 0,39 | 0,39 |
| Paraphénylénediamine (base d'oxydation additionnelle) | 0,162 | - | 0,162 | - | - | - |
| Para-aminophénol (base d'oxydation additionnelle) | - | 0,163 | - | - | - | - |
| 3-méthyl-4-amino phénol (base d'oxydation additionnelle) | - | - | - | 0,184 | - | - |
| Dichlorhydrate de 2-(β-hydroxyéthyl)-paraphénylènediamine (base d'oxydation additionnelle) | - | - | - | - | 0,337 | - |
| Dichlorhydrate de 2,6-diméthylparaphénylènediamine (base d'oxydation additionnelle) | - | - | - | - | - | 0,313 |
| 5-N-(β-hydroxyéthyl)amino 2-méthylphénol (coupleur) | 0,498 | - | - | - | - | - |
| Dichlorhydrate de 2,4-diaminophénoxyéthanol (coupleur) | - | 0,723. | - | - | - | - |
| 1,3-dihydroxy benzène (coupleur) | - | - | 0,33 | - | - | - |
| 5-amino 2-méthyl-phénol (coupleur) | - | - | - | 0,369 | - | - |
| 3-amino phénol **(coupleur)** | - | - | - | - | 0,327 | |
| 6-hydroxybanzomorpholine **(coupleur)** | | | | | | 0,453 |
| Support de teinture commun n°1 | (*) | (*) | (*) | (*) | (*) | (*) |
| Eau déminéralisée q.s.p. | 100 g | 100 g | 100 g | 100 g | 100 g | 100 g |

| | | | | | | |
|---|---|---|---|---|---|---|
| (*) Support de teinture commun n°1: - Alcool éthylique à 96° 18 g - Métabisulfite de sodium en solution aqueuse à 35% a 0,68 g - Sel pentasodique de l'acide diéthylènetriaminopentaacétique 1,1 g - Ammoniaque à 20% de NH₃ 10.0 g | | | | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris, naturels ou permanentés, à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue sur cheveux naturels** | **Nuance obtenue sur cheveux permanentés** |
|---|---|---|---|
| 1 | 10 ± 0,2 | Châtain clair irisé violacé | Châtain violine |
| 2 | 10 ± 0,2 | Cendré violacé | Bleu puissant |
| 3 | 10 ± 0,2 | Blond foncé doré irisé | Châtain clair doré irisé |
| 4 | 10 ± 0,2 | Blond irisé violacé | Châtain clair violine cendré |
| 5 | 10 ± 0,2 | Gris | Gris mat puissant |
| 6 | 10 ± 0,2 | Vert doré | Vert clair |

### EXEMPLES 7 à 12 DE TEINTURE EN MILIEU NEUTRE

On a préparé les compositions tinctoriales conformes à l'invention suivantes (teneurs en grammes):

| **EXEMPLE** | **7** | **8** | **9** | **10** | **11** | **12** |
|---|---|---|---|---|---|---|
| 1,8-bis-(2,5-diaminophénoxy)-3,6-dioxaoctane, tétrachlorhydrate, monohydrate | 0,39 | 0,39 | 0,39 | 0,39 | 0,39 | 0,39 |
| Paraphénylènediamine (base d'oxydation additionnelle) | 0,162 | - | 0,162 | - | - | - |
| Para-aminophénol (base d'oxydation additionnelle) | - | 0,163 | - | - | - | - |
| 3-méthyl-4-amino-phénol (base d'oxydation additionnelle) | - | - | - | 0,184 | - | - |
| Dichlorhydrate de 2-(β-hydroxyéthyl)-paraphénylènediamine (base d'oxydation additionnelle) | - | - | - | - | 0,337 | - |
| Dichlorhydrate de 2,6-diméthylparaphénylènediamine (base d'oxydation additionnelle) | - | - | - | - | - | 0,313 |
| 5-N-(β-hydroxyéthyl)amino 2-méthylphénol (coupleur) | 0,498 | - | - | - | - | - |
| Dichlorhydrate de 2,4-diaminophénoxyéthanol (coupleur) | - | 0,723 | - | - | - | - |
| 1,3-dihydroxy-benzène (coupleur) | - | - | 0,33 | - | - | - |
| 5-amino 2-méthyl-phénol (coupleur) | - | - | - | 0,369 | - | - |
| 3-amino-phénol **(coupleur)** | - | - | - | - | 0,327 | - |
| 6-hydroxybenzomorpholine**(coupleur)** | | | | | | 0,453 |
| Support de teinture commun n°2 | (**) | (**) | (**) | (**) | (**) | (**) |
| Eau déminéralisée q.s.p. | 100 g | 100 g | 100 g | 100 g | 100 g | 100 9 |

| | | | | | | |
|---|---|---|---|---|---|---|
| (**) Support de teinture commun n°2 : - Ethanol à 96° 18 g - Tampon K₂HPO₄ /KH₂PO₄ (1,5 M / 1 M) 10 g - Métabisulfite de sodium 0.68 g - Sel pentasodique de l'acide diéthylénetriaminopentaacétique 1,1 g | | | | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris, naturels ou permanentés, à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue sur cheveux naturels** | **Nuance obtenue sur cheveux permanentés** |
|---|---|---|---|
| **7** | 5,7 ± 0,2 | Châtain clair violine | Châtain violine puissant |
| **8** | 5,7 ± 0,2 | Blond foncé cendré | Châtain clair cendré puissant |
| **9** | 5,7 ± 0,2 | Gris cendré puissant | Gris puissant |
| **10** | 5,7 ± 0,2 | Gris cendré | Gris cendré violacé |
| **11** | 5,7 ± 0,2 | Gris bleu | Gris bleu |
| **12** | 5,7 ± 0,2 | Vert doré | Vert |

## Revendications

1. Composition pour la teinture d'oxydation des fibres kératiniques, et en particulier des fibres kératiniques humaines telles que les cheveux, **caractérisée par le fait qu'**elle contient, dans un milieu approprié pour la teinture:
- du 1,8-bis-(2,5-diaminophénoxy)-3,6-dioxaoctane et/ou au moins un de ses sels d'addition avec un acide, à titre de première base d'oxydation ;
- au moins une base d'oxydation additionnelle choisie parmi la paraphénylènediamine, la paratoluylènediamine, la N,N-bis-(β-hydroxyéthyl)-paraphénylènediamine, la 2-(β-hydroxyéthyl)- paraphénylènediamine, la 2,6-diméthyl-paraphénylènediamine, la 2-isopropyl-paraphénylènediamine, la 2-chloro-paraphénylènediamine, la N-phényl- paraphénylènediamine, la 4,4'-diamino-diphénylamine, la N-méthoxyéthyl - paraphénylènediamine, la 2-n-propyl-paraphénylènediamine, le 4-aminophénol, le N-méthyl-4-aminophénol, le 2-hydroxyméthyl 4-aminophénol, le 3-méthyl 4-aminophénol, le 2-aminométhyl - 4-aminophénol, le 2-(β-hydroxyéthylaminométhyl)- 4-aminophénol, le 2-méthoxy-4-aminophénol, le 2-méthoxyméthyl-4-aminophénol
- et au moins un coupleur.

2. Composition selon la revendication 1, **caractérisée par le fait que** le ou les coupleurs sont choisis parmi les métaphénylènediamines, les métaaminophénols, les métadiphénols et les coupleurs hétérocycliques, et leurs sels d'addition avec un acide.

3. Composition selon la revendication 2, **caractérisée par le fait que** le ou les coupleurs sont choisis parmi le 5-amino-2-méthyl-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy- benzène, le 2,4-diamino-1-(β-hydroxyéthyloxy)-benzène, le 2-amino 4-(β-hydroxyéthylamino)-1-méthoxy benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 6-hydroxybenzomorpholine, la 2,6-dihydroxy-4-méthyl-pyridine, le 1-H 3-méthyl-pyrazole-5-one, le 1-phényl-3-méthyl-pyrazole-5-one, et leurs sels d'addition avec un acide.

4. Composition selon l'une quelconque des revendications précédentes,
**caractérisée par le fait que** les sels d'addition avec un acide sont choisis parmi les chlorhydrates, les bromhydrates, les sulfates, les citrates, les succinates, les tartrates, les lactates et les acétates,

5. Composition selon l'une quelconque des revendications précédentes,
**caractérisée par le fait que** le 1,8-bis-(2,5-diaminophénoxy)-3,6-dioxaoctane et/ou le ou ses sels d'addition avec un acide représentent de 0,0005 à 12% en poids du poids total de fa composition tinctoriale.

6. Composition selon la revendication 5, **caractérisée par le fait que** le 1,8-bis-(2,5-diaminophénoxy)-3,6-dioxaoctane et/ou le ou ses sels d'addition avec un acide représentent de 0.005 à 6 % en poids du poids total de la composition tinctoriale.

7. Composition selon l'une quelconque des revendications précédentes,
**caractérisée par le fait que** la ou les bases d'oxydation additionnelles et/ou le ou leurs sels d'addition avec un acide représentent de 0,0005 à 12 % en poids du poids total de la composition tinctoriale.

8. Composition selon la revendication 7, **caractérisée par le fait que** la ou les bases d'oxydation additionnelles et/ou le ou leurs seis d'additian avec un acide représentent de 0,005 à 6 % en poids du poids total de la composition tinctoriale.

9. Composition selon l'une quelconque des revendications précédentes,
**caractérisée par le fait que** le ou les coupleurs représentent de 0,0001 à 10 % en poids du poids total de la composition tinctoriale

10. Composition selon la revendication 9, **caractérisée par le fait que** le ou les coupleurs représentent de 0,005 à 5 % en poids du poids total de la composition tinctoriale.

11. Procédé de teinture d'oxydation des fibres kératiniques *et* en particulier des fibres kératiniques humaines telles que les cheveux **caractérisé par le fait qu'**on applique sur lesdites fibres au moins une composition tinctoriale telle que définie à l'une quelconque des revendications 1 à 10, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement

12. Procédé selon la revendication 11, **caractérisé par le fait que** l'agent oxydant est choisi parmi le peroxyde d'hydrogène, le peroxyde d'urée, les bromates de métaux alcalins, les persels, et les enzymes.

13. Dispositif à plusieurs compartiments, ou "kit" de teinture à plusieurs compartiments, dont un premier compartiment renferme une composition tinctoriale définie à l'une quelconque des revendications 1 à 10 et un second compartiment renferme une composition oxydante.

## Patentansprüche

1. Zusammensetzung zum oxidativen Färben von Keratinfasern und insbesondere menschlichen Keratinfasern, wie dem Haar, **dadurch gekennzeichnet, daß** sie in einem zum Färben geeigneten Medium enthält:
- 1,8-Bis(2,5-diaminophenoxy)-3,6-dioxaoctan und/oder mindestens eines seiner Additionssalze mit einer Säure als erste Oxidationsbase,
- mindestens eine zusätzliche Oxidationsbase, die ausgewählt ist unter: p-Phenylendiamin, p-Toluylendiamin, N,N-Bis(β-hydroxyethyl)-p-phenylendiamin, 2-(ß-Hydroxyethyl)-p-phenylendiamin, 2,6-Dimethyl-p-phenylendiamin, 2-Isopropyl-p-phenylendiamin, 2-Chlor-p-phenylendiamin, N-Phenyl-p-phenylendiamin, 4,4'-Diamino-diphenylamin, N-Methoxyethyl-p-phenylendiamin, 2-n-Propyl-p-phenylendiamin, 4-Aminophenol, N-Methyl-4-aminophenol, 2-Hydroxymethyl-4-aminophenol, 3-Methyl-4-aminophenol, 2-Aminomethyl-4-aminophenol, 2-(β-Hydroxyethylaminomethyl)-4-aminophenol, 2-Methoxy-4-aminophenol, und
- mindestens einen Kuppler.

2. Zusammensetzung nach Anspruch 1, **dadurch gekennzeichnet, daß** der oder die Kuppler unter den m-Phenylendiaminen, m-Aminophenolen, m-Dihydroxybenzolen und heterocyclischen Kupplern und deren Additionssalzen mit einer Säure ausgewählt sind.

3. Zusammensetzung nach Anspruch 2, **dadurch gekennzeichnet, daß** der oder die Kuppler unter 5-Amino-2-methyl-phenol, 5-N-(β-Hydroxyethyl)-amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(β-hydroxyethyloxy)-benzol, 2-Amino-4-(β-hydroxyethylamino)-1-methoxy-benzol, 1,3-Diaminobenzol, 1,3-Bis(2,4-diaminophenoxy)-propan, Sesamol, α-Naphthol, 6-Hydroxyindol, 4-Hydroxyindol, 4-Hydroxy-N-methyl-indol, 6-Hydroxyindolin, 6-Hydroxy-benzomorpholin, 2,6-Dihydroxy-4-methyl-pyridin, 1-H-3-Methyl-pyrazol-5-on, 1-Phenyl-3-methyl-pyrazol-5-on und den Additionssalzen dieser Verbindungen mit einer Säure ausgewählt sind.

4. Zusammensetzung nach einem der vorhergehenden Ansprüche, **dadurch gekennzeichnet, daß** die Additionssalze mit einer Säure unter den Hydrochloriden, Hydrobromiden, Sulfaten, Citraten, Succinaten, Tartraten, Lactaten und Acetaten ausgewählt sind.

5. Zusammensetzung nach einem der vorhergehenden Ansprüche, **dadurch gekennzeichnet, daß** das 1,8-Bis(2,5-diaminophenoxy)-3,6-dioxaoctan und/oder sein(e) Additionssalz(e) mit einer Säure 0,0005 bis 12 Gew.-% des Gesamtgewichts der Farbmittelzusammensetzung ausmachen.

6. Zusammensetzung nach Anspruch 5, **dadurch gekennzeichnet, daß** das 1,8-Bis(2,5-diaminophenoxy)-3,6-dioxaoctan und/oder sein(e) Additionssalz(e) mit einer Säure 0,005 bis 6 Gew.-% des Gesamtgewichts der Farbmittelzusammensetzung ausmachen.

7. Zusammensetzung nach einem der vorhergehenden Ansprüche, **dadurch gekennzeichnet, daß** die zusätzliche(n) Oxidationsbase(n) und/oder ihr(e) Additionssalz(e) mit einer Säure 0,0005 bis 12 Gew.-% des Gesamtgewichts der Farbmittelzusammensetzung ausmachen.

8. Zusammensetzung nach Anspruch 7, **dadurch gekennzeichnet, daß** die zusätzliche(n) Oxidationsbase(n) und/oder ihr(e) Additionssalz(e) mit einer Säure 0,005 bis 6 Gew.-% des Gesamtgewichts der Farbmittelzusammensetzung ausmachen.

9. Zusammensetzung nach einem der vorhergehenden Ansprüche, **dadurch gekennzeichnet, daß** der oder die Kuppler 0,0001 bis 10 Gew.-% des Gesamtgewichts der Farbmittelzusammensetzung ausmachen.

10. Zusammensetzung nach Anspruch 9, **dadurch gekennzeichnet, daß** der oder die Kuppler 0,005 bis 5 Gew.-% des Gesamtgewichts der Farbmittelzusammensetzung ausmachen.

11. Verfahren zum oxidativen Färben von Keratinfasern und insbesondere menschlichen Keratinfasern, wie dem Haar, **dadurch gekennzeichnet, daß** auf die Fasern eine Farbmittelzusammensetzung nach einem der Ansprüche 1 bis 10 aufgetragen und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

12. Verfahren nach Anspruch 11, **dadurch gekennzeichnet, daß** das Oxidationsmittel unter Wasserstoffperoxid, Harnstoffperoxid, Alkalimetallbromaten, Salzen von Persäuren und Enzymen ausgewählt ist.

13. Vorrichtung mit mehreren Abteilungen oder "Kit" zum Färben mit mehreren Abteilungen, wobei eine erste Abteilung die Farbmittelzusammensetzung nach einem der Ansprüche 1 bis 10 und eine zweite Abteilung eine oxidierende Zusammensetzung enthält.

## Claims

1. Composition for the oxidation dyeing of keratin fibres, and in particular human keratin fibres such as the hair, **characterized in that** it contains, in a medium which is suitable for dyeing:
- 1,8-bis(2,5-diaminophenoxy)-3,6-dioxaoctane and/or at least one of the addition salts thereof with an acid, as first oxidation base;
- at least one additional oxidation base chosen from para-phenylenediamine, para-tolylenediamine, N,N-bis-(β-hydroxyethyl)-para-phenylenediamine, 2-(β-hydroxyethyl)-para-phenylenediamine, 2,6-dimethyl-paraphenylenediamine, 2-isopropyl-para-phenylenediamine, 2-chloro-para-phenylenediamine, N-phenyl-paraphenylenediamine, 4,4'-diaminodiphenylamine, N-methoxyethyl-para-phenylenediamine, 2-n-propyl-paraphenylenediamine, 4-aminophenol, N-methyl-4-aminophenol, 2-hydroxymethyl-4-aminophenol, 3-methyl-4-aminophenol, 2-aminomethyl-4-aminophenol, 2-(β-hydroxyethylaminomethyl)-4-aminophenol, 2-methoxy-4-aminophenol, 2-methoxymethyl-4-aminophenol
- and at least one coupler.

2. Composition according to Claim 1, **characterized in that** the coupler(s) is (are) chosen from meta-phenylenediamines, meta-aminophenols, meta-diphenols and heterocyclic couplers, and the addition salts thereof with an acid.

3. Composition according to Claim 2, **characterized in that** the coupler(s) is (are) chosen from 5-amino-2-methylphenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 6-hydroxybenzomorpholine, 2,6-dihydroxy-4-methylpyridine, 1-H-3-methylpyrazol-5-one and 1-phenyl-3-methylpyrazol-5-one, and the addition salts thereof with an acid.

4. Composition according to any one of the preceding claims, **characterized in that** the addition salts with an acid are chosen from the hydrochlorides, hydrobromides, sulphates, citrates, succinates, tartrates, lactates and acetates.

5. Composition according to any one of the preceding claims, **characterized in that** the 1,8-bis(2,5-diaminophenoxy)-3,6-dioxaoctane and/or the addition salt(s) thereof with an acid represent(s) from 0.0005 to 12% by weight relative to the total weight of the dye composition.

6. Composition according to Claim 5, **characterized in that** the 1,8-bis(2,5-diaminophenoxy)-3,6-dioxaoctane and/or the addition salt(s) thereof with an acid represent(s) from 0.005 to 6% by weight relative to the total weight of the dye composition.

7. Composition according to any one of the preceding claims, **characterized in that** the additional oxidation base(s) and/or the addition salt(s) thereof with an acid represent(s) from 0.0005 to 12% by weight relative to the total weight of the dye composition.

8. Composition according to Claim 7, **characterized in that** the additional oxidation base(s) and/or the addition salt(s) thereof with an acid represent(s) from 0.005 to 6% by weight relative to the total weight of the dye composition.

9. Composition according to any one of the preceding claims, **characterized in that** the coupler(s) represent(s) from 0.0001 to 10% by weight relative to the total weight of the dye composition.

10. Composition according to Claim 9, **characterized in that** the coupler(s) represent(s) from 0.005 to 5% by weight relative to the total weight of the dye composition.

11. Process for the oxidation dyeing of keratin fibres, and in particular human keratin fibres such as the hair, **characterized in that** at least one dye composition as defined in any one of Claims 1 to 10 is applied to the said fibres and the colour is developed at acidic, neutral or alkaline pH with the aid of an oxidizing agent which is added to the dye composition just at the time of use, or which is present in an oxidizing composition that is applied simultaneously or sequentially.

12. Process according to Claim 11, **characterized in that** the oxidizing agent is chosen from hydrogen peroxide, urea peroxide, alkali metal bromates, persalts and enzymes.

13. Multi-compartment dyeing device or multi-compartment dyeing "kit", a first compartment of which contains a dye composition defined in any one of Claims 1 to 10, and a second compartment of which contains an oxidizing composition.
